# EUROPÄISCHE PATENTSCHRIFT

(11) **EP 2 524 670 B1**
(45) Veröffentlichungstag und Bekanntmachung des Hinweises auf die Patenterteilung: **01.07.2020**
(21) Anmeldenummer: 12003961.5
(22) Anmeldetag: 21.05.2012
(51) Int. Cl.: A61F 2/00

(54) **Dreidimensionales Herniennetz**
Three-dimensional hernia mesh
Maillage tridimensionnel pour hernie

(30) Priorität: 19.05.2011 DE 102011102039
(43) Veröffentlichungstag der Anmeldung: 21.11.2012
(73) Patentinhaber: FEG Textiltechnik Forschungs- und Entwicklungsgesellschaft MbH, 52070 Aachen (DE)
(72) Erfinder: Löhde, Eckhard, 14129 Berlin (DE)
(74) Vertreter: Gottschald, Jan

(56) Entgegenhaltungen:
- EP-A2- 0 898 944
- EP-B1- 1 421 916
- WO-A1-02/22047
- WO-A1-2011/126724
- US-A1- 2003 212 462
- US-A1- 2004 143 344

## Beschreibung

Die Erfindung betrifft ein Implantat gemäß dem Oberbegriff von Anspruch 1.

Das in Rede stehende Implantat dient zum Verschließen von Wunden oder offenen Muskelspalten oder dergleichen des menschlichen Körpers, und kann zum Beispiel zum Verschluss von Defekten der inneren und äußeren Bauchwand (Hernien), bei Zwerchfell-Brüchen oder dergleichen eingesetzt werden.

Im Stand der Technik ist z. B. aus US 6,113,623 ein Implantat bzw. eine Prothese zum Verschließen einer Hernie bekannt. Dieses Implantat besteht aus mehreren textilen Lappen bzw. Flächenelementen, die miteinander in einem mittleren Verbindungsbereich verbunden sind und somit eine dreidimensionale Struktur ausbilden. Dieses Implantat dient zum Verschließen eines Risses in der Bauchwand eines menschlichen Körpers, wobei die Größe der flächigen Lappen an den Riss bzw. die Ausstülpung im Bereich der Bauchwand angepasst ist.

Die EP 0 898 944 A2 beschreibt eine Prothese zur Hernienbehandlung, welche einen Kanal aus einem Flächenmaterial zum Einführen in die Hernie aufweist. Der Kanal weist an jeweils gegenüberliegenden Enden einen Kragen und eine Basis auf, welche Basis innerhalb der Hernie angeordnet werden soll.

Das bekannte Implantat gemäß EP 1 421 916 B1, von dem die vorliegende Erfindung ausgeht, weist ebenfalls eine dreidimensionale Struktur auf, die aus einer Mehrzahl von flächigen Lagen bzw. Ebenen aus einem textilen netzförmigen Material gebildet sind. Dieses Implantat wird in der Mitte einer Wunde einer Bauchwand-Hernie verwendet, um den Bauchwandverschluss zu verstärken. Ein solches Implantat weist eine erste Lage auf, die sich für eine senkrechte Anordnung zwischen den Rändern der Aponeurose der zu verschließenden Wunde eignet. Ferner weist dieses Implantat zumindest eine weitere Lage auf, die so angeordnet ist, dass sie mit der ersten Lage einen Flächenwinkel bildet. Diese weitere Lage eignet sich für eine die Ränder der zu verbindenden Aponeurose waagerecht überlappende Anordnung, wobei diese Anordnung die Widerstandskraft einer Naht, mit der die offene Wunde vernäht wird, erhöhen soll.

Falls die bekannten Implantate aus einem textilen Material gebildet sind, besteht an einer äußeren Kante des Implantats die Gefahr von Ausfransungen oder dergleichen. Falls es in einem offenen Wundbereich des menschlichen Körpers zu einem Kontakt zwischen solchen Ausfransungen an einer äußeren Kante des Implantats und dem menschlichen Gewebe kommt, so kann das menschliche Gewebe dadurch geschädigt werden, z.B. durch Mikrorisse. Solche Mikrorisse können dann zu nachteiligen Entzündungen des Körpergewebes bzw. zu Wundkomplikationen führen.

Der Erfindung liegt das Problem zugrunde, das bekannte Implantat derart auszugestalten und weiterzubilden, dass ein Hineinbringen bzw. Einsetzen des Implantats in eine Wunde des menschlichen Körpers mit größerer Sicherheit und ohne Wundkomplikationen erfolgen kann.

Das obige Problem wird durch ein Implantat mit den Merkmalen von Anspruch 1 gelöst. Vorteilhafte Weiterbildungen der Erfindung sind in den abhängigen Ansprüchen definiert.

Wesentlich für das erfindungsgemäße Implantat ist die Überlegung, dass eine freie äußere Kante eines flächigen Lagenelementes, das Teil des Implantats ist, in zumindest einem Abschnitt einen abgeschlossenen Rand aufweist. Dies bedeutet, dass der Randbereich dieser freien äußeren Kante einen homogenen bzw. einheitlichen Verlauf hat, ohne dass z.B. einzelne Textilfilamente bzw. Fäden, aus denen das Lagenelement zumindest teilweise bestehen kann, freiliegen und z. B. nach außen abragen. Entsprechend hat diese freie äußere Kante des flächigen Lagenelements in zumindest einem bestimmten Abschnitt eine "gute" und somit einheitlich definierte Struktur, ohne dass die Gefahr von Ausfransungen oder dergleichen besteht. Falls beim Einsetzen des Implantats in eine Wunde des menschlichen Körpers die freie äußere Kante des flächigen Lagenelementes in Kontakt mit Körpergewebe gelangt, ist wegen des abgeschlossenen Rands der äußeren Kante eine Schädigung des Körpergewebes z.B. durch Mikrorisse vermindert oder gar ausgeschlossen. Entsprechend ergeben sich bei einer Verwendung des erfindungsgemäßen Implantats weniger oder bestenfalls gar keine Wundkomplikationen.

Die Ausbildung des abgeschlossenen Randes der äußeren freien Kante erfolgt vorschlagsgemäß dadurch, dass das Material des Lagenelements im Bereich der äußeren Kannte umgeschlagen ist. Hierzu ist das Material des Lagenelements ausreichend flexibel ausgebildet, so dass ein Umschlagen ohne weiteres möglich ist. Ein Fixieren des umgeschlagenen Teils erfolgt in bekannter Weise, z.B. durch Kleben oder Thermofixieren. Alternativ oder ergänzend zu dem Umschlagen der freien äußeren Kante ist es auch möglich, die äußere Kante des Lagenelements mit einer Silikonschicht zu versehen, wobei dann der abgeschlossene Rand der äußeren Kante aus der Silikonschicht gebildet ist. Bei beiden der genannten Möglichkeiten ist gewährleistet, dass die freie äußere Kante an ihrem Randbereich nicht ausfranst und einen homogen verlaufenden Rand beibehält. Nach einer weiteren Ausführungsform ist es auch möglich, das Umschlagen der freien äußeren Kante und das Aufbringen einer Silikonschicht im Bereich dieser freien äußeren Kante miteinander zu kombinieren. Hierbei kann die Silikonschicht dazu dienen, den umgeschlagenen Teil des Materials des Lagenelements zu fixieren, so dass ein Zurückklappen des umgeschlagenen Teils der freien äußeren Kante und/oder deren Ausfransen wirkungsvoll verhindert wird.

Ein weiter verbesserter Schutz des menschlichen Gewebes bei einem evtl. Kontakt mit dem Implantat lässt sich dadurch erreichen, dass die freie äußere Kante in zumindest einem Abschnitt im Querschnitt gerundet ausgebildet ist (Anspruch 2). Da das Lagenelement an seiner äußeren Kante umgeschlagen ist, resultiert durch das Umschlagen der äußeren Kante des Lagenelements dort kein scharfkantiger Knick, sondern stattdessen ein Bereich mit einer gerundeten Kontur, die das menschliche Gewebe bei einem Kontakt mit dem Implantat schont. In gleicher Weise kann das Aufbringen einer Silikonschicht zu einer Rundung der freien äußeren Kante im Querschnitt führen.

Eine besonders umfassende Schonung des Körpergewebes bei einem evtl. Kontakt mit dem Implantat wird dadurch erreicht, dass die freie äußere Kante zumindest eines Lagenelementes in ihrer gesamten Länge einen abgeschlossenen Rand aufweist, und/oder dass bei einer Mehrzahl von flächigen Lagenelementen alle diese Lagenelemente zumindest eine freie äußere Kante aufweisen, die wie vorstehend erläutert einen abgeschlossenen Rand aufweisen.

Eine besonders kostengünstige Herstellung des Implantats wird dadurch gewährleistet, dass ein flächiges Material durch eine geeignete Falttechnik zu dem Implantat in seinem Endzustand ausgebildet wird. Besonders vorteilhaft hierbei ist, wenn das flächige Material einstückig ist, so dass das Implantat nicht aus mehreren zu verbindenden Teilen besteht (Anspruch 4).

In vorteilhafter Weiterbildung kann das flächige Material, aus dem das Implantat gefaltet wird, in seinem Ausgangszustand aus einem Endlos-Schlauch bestehen, so dass das Implantat im gefalteten Zustand eine Mehrzahl von Lagenelementen mit jeweils zumindest einer freien äußeren Kante aufweist, die umgeschlagen ist und dadurch einen abgeschlossenen Rand aufweist. Hierdurch ist die Gefahr der Bildung von Mikrorissen im Körpergewebe weiter vermindert, so dass die Wahrscheinlichkeit von Wundkomplikationen geringer ist.

Durch die Formgebung des Implantats in Form einer dreidimensionalen Struktur kann eine wirkungsvolle Anpassung an eine Hernie o. dgl. des menschlichen Körpers erzielt werden. Hierzu kann zumindest ein flächiges Lagenelement des Implantats von dessen übrigen Teil flügelartig in Form eines Flügelstegs abragen. Zweckmäßigerweise sind zwei zueinander entgegengesetzte Flügelstege vorgesehen, die zwischen sich einen Mittelsteg einschließen und sich seitlich weg davon erstrecken. In Anpassung an einen jeweiligen Wundbereich des menschlichen Körpers kann das Implantat als T-Profil oder als H-Profil ausgebildet sein. Die Einbringung des Implantats in einen Wundbereich des menschlichen Körpers wird dadurch erleichtert, dass zumindest zwei Lagenelemente relativ zueinander beweglich sind. Eine weiter verbesserte Möglichkeit des Einbringens in einen Wundbereich des menschlichen Körpers resultiert daraus, dass zumindest ein Flügelsteg angrenzend an eine Stirnseite des Implantats gepfeilt ausgebildet ist.

Im Folgenden wird die Erfindung anhand von Ausführungsbeispielen und Zeichnungen näher erläutert.
- Figur 1: zeigt eine Perspektivansicht eines erfindungsgemäßen Implantats nach einer ersten Ausführungsform,
- Figur 2: eine Ansicht von vorne bzw. hinten des Implantats von Figur 1,
- Figur 3: eine Ansicht von oben bzw. unten des Implantats von Figur 1,
- Figur 4: eine Ansicht von rechts auf das Implantat von Figur 1,
- Figur 5: einen sogenannten Faltplan für ein flächiges Material, zur Herstellung des Implantats von Figur 1,
- Figur 6: eine Ansicht auf eine vordere bzw. hintere Stirnseite eines Implantats nach einer weiteren Ausführungsform des erfindungsgemäßen Implantats,
- Figur 7 - 11: eine Schrittabfolge zum Einbringen des erfindungsgemäßen Implantats in eine offene Wunde eines menschlichen Körpers,
- Figur 12: eine Querschnittansicht der Wunde gemäß der Figuren 7 - 11, wenn das Implantat von Figur 1 darin vollständig eingebracht und vernäht ist,
- Figur 13 - 14: Draufsichten auf ein erfindungsgemäßes Implantat nach wieteren Ausführungsformen des erfindungsgemäßen Implantats,
- Figur 15 - 16: Ansichten auf eine vordere bzw. hintere Stirnseite von weiteren Ausführungsformen des erfindungsgemäßen Implantats.
- Figur 17: einen sogenannten Faltplan für ein flächiges Material, zur Herstellung einer weiteren Ausführungsform eines erfindungsgemäßen Implantats, und
- Figur 18: eine Ansicht von unten auf ein Implantat, das nach dem Faltplan gemäß Figur 17 hergestellt ist.

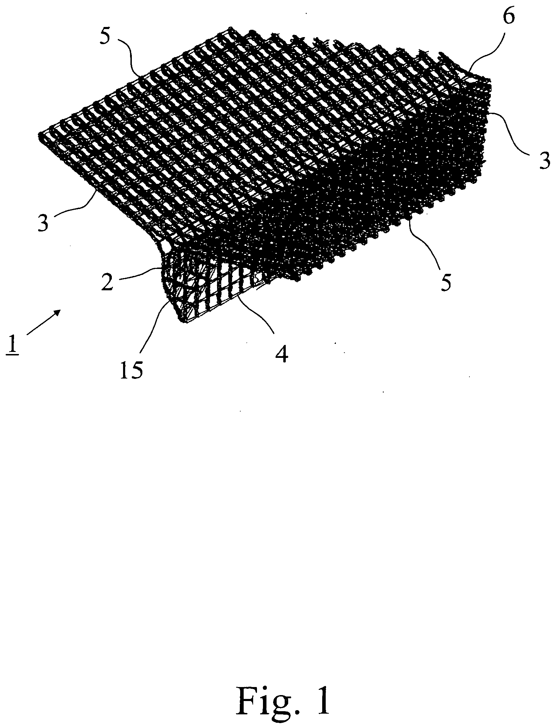

In den Figuren 1 - 4 ist eine erste Ausführungsform eines erfindungsgemäßen Implantats 1 gezeigt. Das Implantat 1 weist eine dreidimensionale Struktur auf, und besteht aus einem vertikal verlaufenden Mittelsteg 2, von dem sich zwei Flügelstege 3 seitlich weg erstrecken.

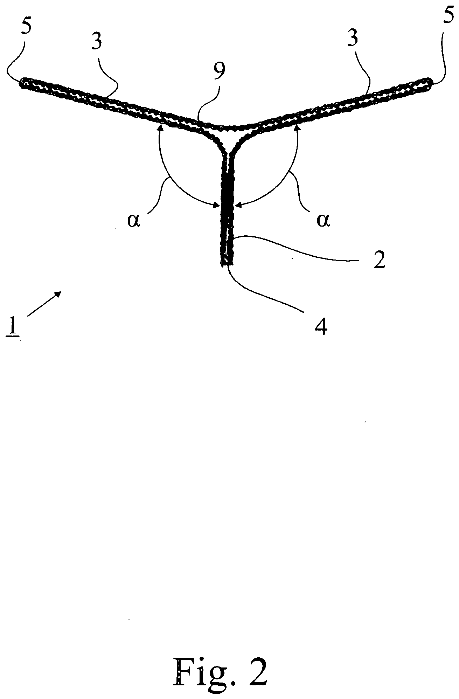

Figur 1 zeigt das Implantat 1 in einer Perspektivansicht von schräg hinten, wobei Figur 2 eine Ansicht auf eine vordere bzw. hintere Stirnseite des Implantats 1 darstellt. Das Implantat 1 ist vorzugsweise aus einem textilen Material hergestellt, das eine netzartige Struktur von Einzelfilamenten aufweisen kann, wie aus Fig. 1 ersichtlich. Der Mittelsteg 2 und die beiden Flügelstege 3 bilden jeweils flächige Lagenelemente, die in einen vorbestimmten Winkel relativ zueinander positioniert sind. Die Stirnseiten-Ansicht von Figur 2 verdeutlicht, dass die beiden Flügelstege 3 jeweils mit dem Mittelsteg 2 einen Winkel a einschließen, der geringfügig größer als 90 ° ist. Das Implantat 1 ist in der Darstellung von Fig. 1 und Fig. 2 in einem Endzustand gezeigt, bei dem die beiden Flügelstege 3 mit dem Winkel a bezüglich des Mittelstegs 2 positioniert sind. Jedoch ist das Material, aus dem das Implantat 1 hergestellt ist, insoweit elastisch, dass die Flügelstege 3 in ihrer Position relativ zum Mittelsteg 2 verändert werden können, wobei der Winkel a größer oder kleiner wird.

Die Stirnseitenansicht von Fig. 2 verdeutlicht, dass der Mittelsteg 2 eine untere freie äußere Kante 4 aufweist. Die beiden Flügelstege 3 weisen ebenfalls jeweils eine freie äußere Kante 5 auf, wobei das Material der Flügelstege 3 an dieser Kante umgeschlagen ist und somit einen abgeschlossenen Rand aufweist. Entsprechend liegen an diesem abgeschlossenen Rand keine Faserenden, Einzelfilamente o. dgl. frei. Durch das Umschlagen der Flügelstege 3 an der freien äußeren Kante 5 kann der abgeschlossene Rand im Querschnitt gerundet ausgebildet sein.

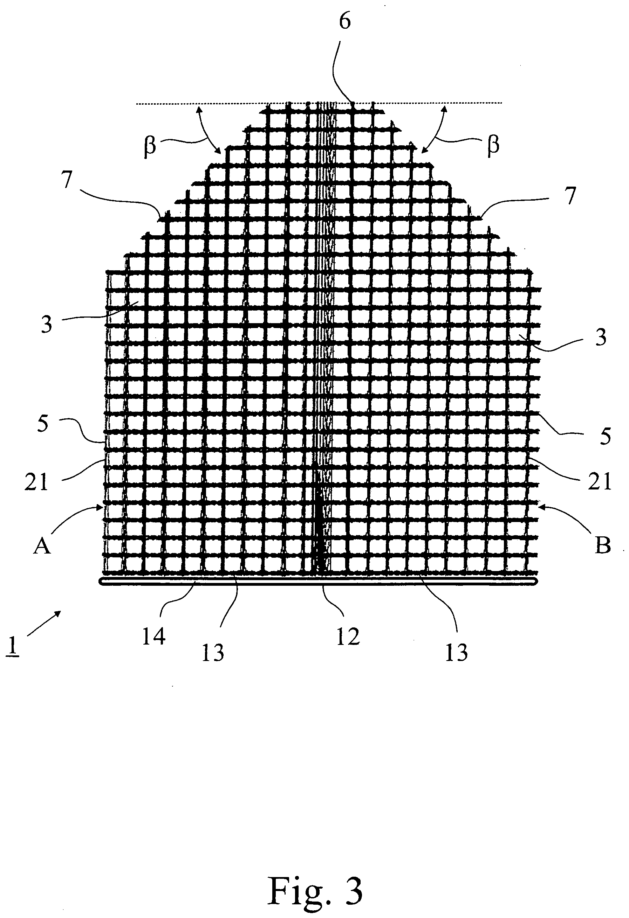

Figur 3 zeigt eine Draufsicht auf das Implantat 1 von Figur 1. Hierin ist ersichtlich, dass die beiden Flügelstege 3 in einem an eine vordere Stirnseite 6 des Implantats 1 angrenzenden Abschnitt 7 mit einer Pfeilung versehen sind. Unter dem Begriff Pfeilung ist zu verstehen, dass die äußere Kante 5 der Flügelstege 3 nicht durchgehend parallel zum Mittelsteg 2 verläuft, sondern in dem Abschnitt 7 angrenzend an die vordere Stirnseite 6 abgeschrägt verläuft. Der Winkel, den dieser abgeschrägte Abschnitt 7 der jeweiligen Flügelstege 3 mit einer parallel zur vorderen Stirnseite 6 verlaufenden Ebene einschließt, ist in Figur 3 mit "β" bezeichnet. Der Hintergrund und Zweck der Pfeilung 7 der beiden Flügelstege 3 ist nachstehend noch im Detail erläutert.

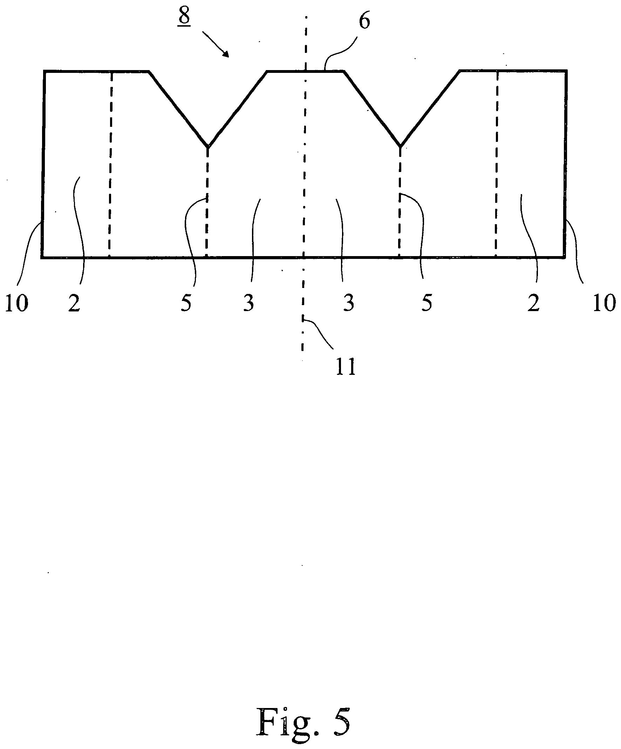

Das Implantat 1 lässt sich aus einem Material 8 (Fig. 5) herstellen, das vorzugsweise aus einem netzartigen Textil besteht. Ein solches netzartiges Textil kann in seinem Ausgangszustand aus einer durchgehenden zweidimensionalen Fläche bestehen, die sich mittels einer vorbestimmten Falttechnik zu dem dreidimensionalen Implantat 1 gemäß der Darstellung von Figur 1 umformen bzw. falten lässt.

Figur 5 zeigt eine Draufsicht auf das flächige Material 8 in seinem Ausgangszustand. Die gestrichelten Linien deuten dabei die Körperkanten an, entlang derer das Material 8 zu falten ist. Auf Grundlage des in Figur 5 gezeigten Materials 8 ergibt sich durch das Falten entlang der gestrichelten Linien das Implantat, wie es z. B. in Figur 2 gezeigt ist. Im fertigen Endzustand des Implantats weist eine dem Mittelsteg 2 entgegengesetzte Oberseite 9 der beiden Flügelstege 3 einen durchgehenden Verlauf auf, so dass diese Oberseite 9 im Bereich des Mittelstegs 2 keinen Spalt oder dergleichen bildet. Die beiden äußeren Kanten 10 des Materials 8, die parallel zur Symmetrielinie 11 (vgl. Figur 5) verlaufen, sind an der unteren äußeren Kante 4 des Mittelstegs 2 zusammengefügt (vgl. Fig. 2) und dort mittels Thermoformen, Kleben oder dergleichen miteinander fixiert. Entsprechend ist ein Ausfransen des Mittelstegs 2 an seiner unteren äußeren Kante 4 unterbunden.

Wie vorstehend erläutert, wird durch das Falten des Materials 8 gemäß Figur 5 das Implantat 1 in seinem Endzustand hergestellt, wie es z. B. in Figur 1 gezeigt ist. Zum besseren Verständnis sind in Figur 5 die wesentlichen Elemente des Implantats 1, nämlich der Mittelsteg 2 und die Flügelstege 3 mit ihren jeweiligen äußeren Kanten 5 mit den dazugehörigen Bezugszeichen in Figur 5 gekennzeichnet.

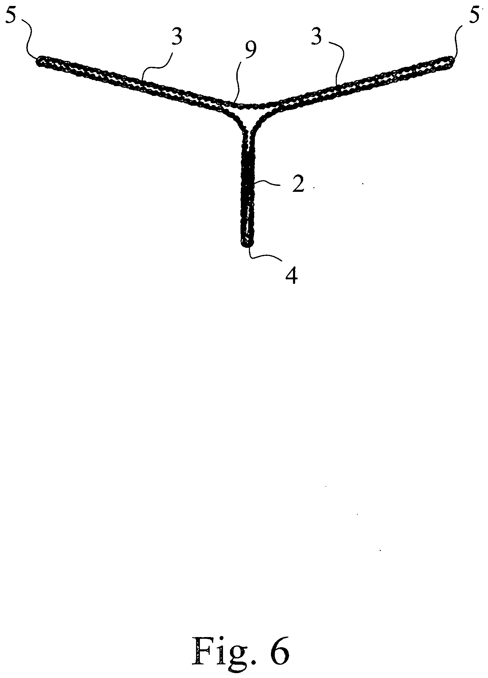

Nach einer weiteren Ausführungsform ist es auch möglich, das Implantat 1 aus einem sogenannten Endlos-Schlauch zu falten. Ausgehend von dem in Figur 5 gezeigten Material 8 ist ein solcher Endlos-Schlauch dahingehend zu verstehen, dass die beiden äußeren Kanten 10 des flächigen Materials 8 miteinander verbunden sind, so dass das Material 8 einen durchgehenden Schlauch ohne eine freie äußere Kante parallel zur Symmetrielinie 11 bildet. Aus einem solchen Schlauch lässt sich in gleicher Weise wie vorstehend erläutert ein dreidimensionales Implantat 1 falten, das in seinem Endzustand z. B. in Figur 1 gezeigt ist. Bedingt dadurch, dass die beiden äußeren Kanten 10 an dem Endlos-Schlauch nunmehr miteinander einstückig verbunden sind, ist die resultierende untere freie äußere Kante 4 des Implantats 1 in seinem Endzustand ebenfalls umgeschlagen und weist dadurch einen abgeschlossenen Rand auf, der vorzugsweise im Querschnitt auch gerundet ausgebildet sein kann. Die Ansicht von hinten gemäß Figur 6 verdeutlicht diese Ausführungsform. Es ist zu erkennen, dass sowohl die beiden äußeren Kanten 6 der jeweiligen Flügelstege 3 als auch die untere äußere Kante 4 des Mittelstegs 2 im Querschnitt gerundet sind.

Der in Fig. 5 gezeigte Faltplan zum Falten des Implantats 1 gemäß der Darstellung von Fig. 1 bzw. zu dessen Herstellung ist nur beispielhaft zu verstehen. Entsprechend kann das Implantat 1 auch in anderer geeigneter Weise aus einem flächigen Material gefaltet werden, um die dreidimensionale Struktur des Implantats 1 zu erzielen. Beispielsweise ist es auch möglich, die Flügelstege 3 im Bereich ihrer gepfeilten Abschnitte 7 umzuschlagen, um auch dort einen abgeschlossenen Rand der freien äußeren Kante 5 zu gewährleisten und ein Ausfransen zu verhindern. Weiter alternativ ist es auch möglich, das Implantat aus einzelnen Elementen herzustellen, die in geeigneter Weise zu der gezeigten dreidimensionalen Struktur zusammengefügt werden.

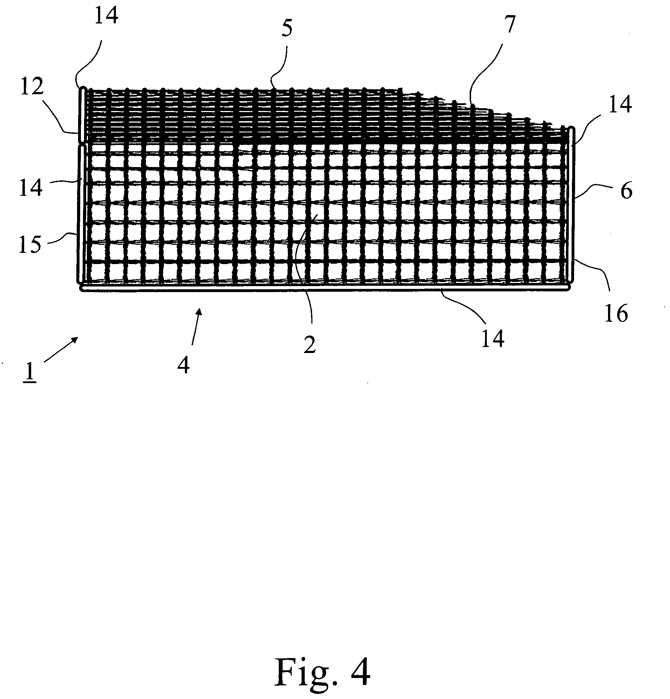

Im Hinblick auf einen gewünschten abgeschlossenen Rand einer freien äußeren Kante des Mittelstegs 2 und/oder der Flügelstege 3, ist es grundsätzlich auch möglich, zumindest einen Abschnitt einer jeweiligen freien äußeren Kante mit einer Silikonschicht zu versehen. Hierbei wird dann der abgeschlossene Rand der freien äußeren Kante aus der Silikonschicht gebildet, die die äußere Kante nach außen hin begrenzt. In gleicher Weise wie ein Umschlagen der freien äußeren Kante verhindert die Silikonschicht ein Ausfransen der freien äußeren Kante oder die Bildung von ähnlichen Unregelmäßigkeiten, wobei die Silikonschicht auch zu einer Rundung der freien äußeren Kante im Querschnitt führen kann. Es ist möglich, eine Silikonschicht an der entlang einer hinteren Stirnseite 12 des Implantats 1 verlaufenden hinteren äußeren Kante 13 der jeweiligen Flügelstege 3 aufzubringen, wobei diese Silikonschicht mit dem Bezugszeichen 14 bezeichnet ist (Fig. 3). In gleicher Weise ist es möglich, den Mittelsteg 2 entlang seiner unteren äußeren Kante 4 mit einer Silikonschicht 14 zu versehen (vgl. Fig. 4). Schließlich ist es auch möglich, eine Silikonschicht an einer hinteren äußeren Kante 15 des Mittelstegs 2, die an der hinteren Stirnseite 12 des Implantats 1 verläuft, aufzubringen. In gleicher Weise kann eine Silikonschicht auch an einer vorderen äußeren Kante 16 des Mittelstegs 2 im Bereich der vorderen Stirnseite 6 des Implantats aufgebracht werden. Die Seitenansicht von Figur 4 verdeutlicht dies, wobei eine Silikonschicht vereinfacht dargestellt und wiederum mit dem Bezugszeichen 14 bezeichnet ist.

In bevorzugter Weiterbildung der Erfindung kann eine Silikonschicht auch an den freien äußeren Kanten 5 der jeweiligen Flügelstege 3, und/oder in dem Bereich von deren Pfeilung 7 (vgl. Fig. 3) aufgebracht werden. Ein Aufbringen der Silikonschicht im Bereich der freien äußeren Kanten 5 der Flügelstege 3 empfiehlt sich besonders dann, wenn die Flügelstege 3 nicht durch ein Falten eines Materials gebildet werden.

Das oben erläuterte Implantat 1 dient zur chirurgisch-operativen Verbindung von Muskeln und Faszien im menschlichen Körper, und insbesondere zur Operation von sogenannten Hernien, d. h. von Brüchen oder Rissen im Bereich der Bauchwanddecke. Falls das Implantat 1 in den Wundbereich des Körpergewebes eingebracht ist, befindet sich der Mittelsteg 2 zwischen zwei Wundflächen, wobei die beiden Flügelstege 3 zur Fixierung des Implantats 1 an den dem Bruch benachbarten Gewebepartien dienen.

Das flächige Material 8, aus dem das Implantat 1 gebildet ist, ist aus einem netzförmigen Textil gebildet, wobei die Netzstruktur des Implantats 1 ein optimales Einwachsverhalten in das Körpergewebe gewährleistet und sich zum Vernähen der Wunde sehr gut mit einem Faden verbinden lässt. Das Material 8 ist ausreichend flexibel ausgestaltet, so dass das oben erläuterte Umschlagen an den äußeren Kanten 5 der jeweiligen Flügelstege 3 bzw. das Falten entlang der in Figur 5 gezeigten gestrichelten Linien ohne Weiteres möglich ist. In Verbindung hiermit weist das Material 8 auch eine ausreichende Steifigkeit dahingehend auf, dass das Implantat 1 in seinem Endzustand, z. B. in der dreidimensionalen Struktur gemäß Figur 1, ausreichend formstabil ist. Der Endzustand von Figur 1 ist als eine Grundform zu verstehen, die sich im Verlauf einer Operation an die Wunde des menschlichen Gewebes anpassen lässt, z. B. hinsichtlich des Winkels a zwischen dem Mittelsteg 2 und den jeweiligen Flügelstegen 3.

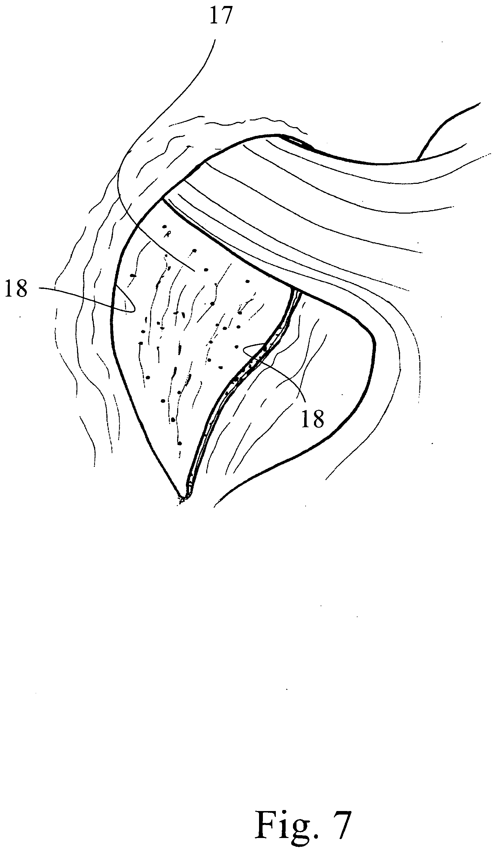

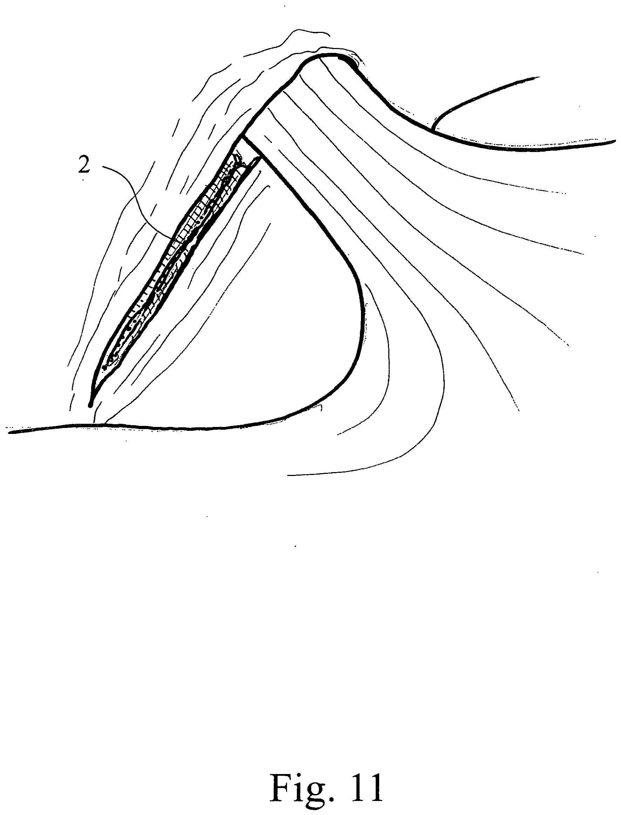

Nachstehend wird unter Bezugnahme auf die Figuren 7 - 12 eine Operation einer offenen Wunde von menschlichem Gewebe unter Verwendung des Implantats 1 erläutert.

Figur 7 zeigt in vereinfachter Weise einen offenen Wundbereich von menschlichem Gewebe, wobei der Wundbereich einen Durchbruch 17 aufweist, mit einander gegenüberliegenden Wundrändern 18. Im Verlauf der Operation wird der Wundbereich 17 mit geeigneten Mitteln offen gehalten bzw. aufgespreizt, wobei anschließend das Implantat 1 mit der vorderen Stirnseite 6 bzw. der Oberseite 9 der Flügelstege 3 voran in den Durchbruch 17 eingebracht wird.

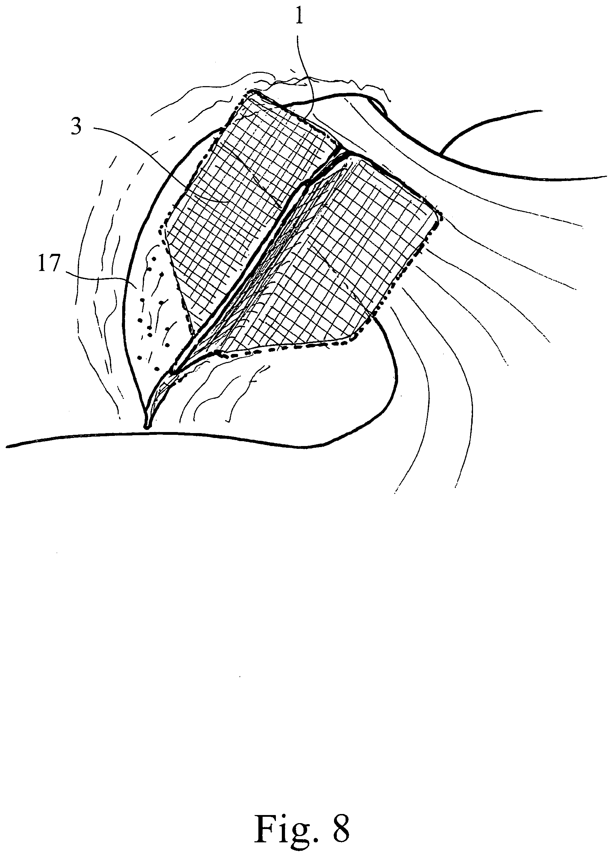

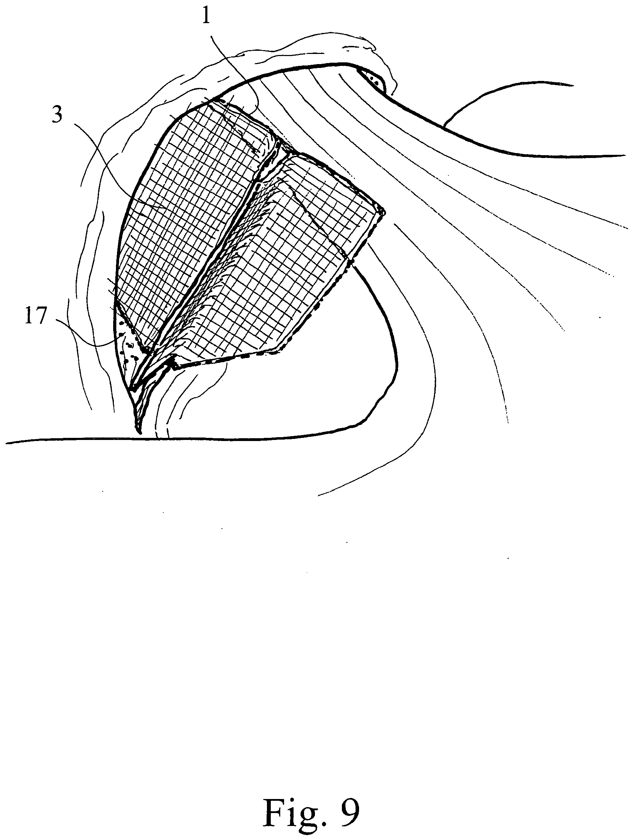

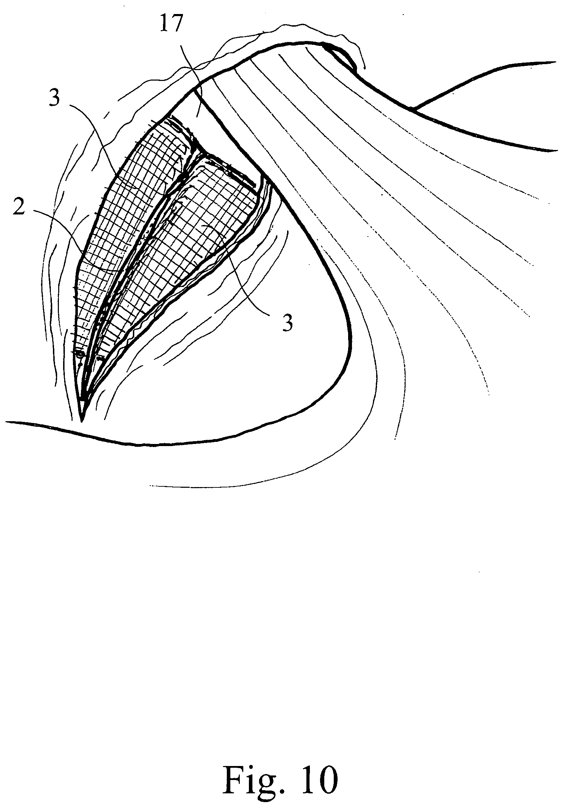

Eine Schrittabfolge eines solchen Einbringens des Implantats 1 in den Durchbruch 17 ist im Verlauf der Figuren 8 bis 11 gezeigt. Insbesondere die Figuren 9 und 10 lassen erkennen, dass die beiden Flügelstege 3 jeweils hinter den Wundrändern 18 im Körperinnern positioniert werden, wobei der Mittelsteg 2 zwischen den gegenüberliegenden Wundrändern 18 des Durchbruchs 17 aus der Wunde herausragt. Das Einbringen des Implantats 1 in den Durchbruch 17 wird wesentlich durch die Pfeilung 7 der Flügelstege 3 erleichtert, weil das Implantat 1 deswegen an seiner vorderen Stirnseite 6, mit der es voran in den Durchbruch 17 eingeführt wird, weniger Platz beansprucht. Das Einbringen des Implantats 1 in den Durchbruch 17 wird des Weiteren dadurch erleichtert, dass die Flügelstege 3 relativ zum Mittelsteg 3 beweglich sind, und sich somit die Neigung der Flügelstege 3 mit ihrem Winkel a bezüglich des Mittelstegs 2 verändern lässt. Die gerundeten äußeren Kanten des Implantats 1, z. B. die äußeren Kanten 6 der beiden Flügelstege 3, führen zu dem Vorteil, dass die Wundränder 18 der Wunde bzw. des Durchbruchs 17 bei einem evtl. Kontakt mit dem Implantat 1 nicht verletzt werden und sich somit keine Mikrorisse oder dergleichen bilden.

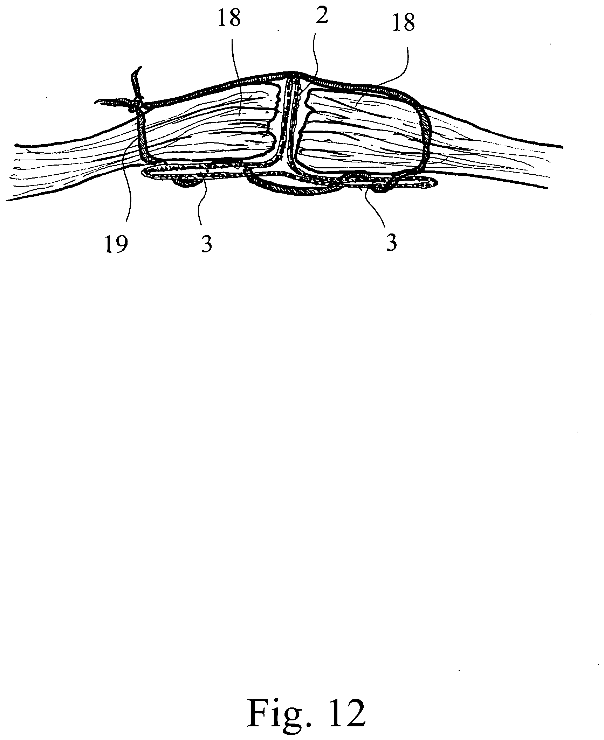

In Figur 12 ist der Wundbereich in einer Querschnittsdarstellung gezeigt, wobei das Implantat 1 vollständig in den Durchbruch 17 eingebracht ist und die gegenüberliegenden Gewebepartien bzw. Wundränder 18 mit dem Implantat 1 vernäht sind. Das Vernähen des Implantats 1 mit dem Körpergewebe ist durch einen geeigneten Faden 19 gewährleistet, der sich sehr gut mit der Netzstruktur des Implantats 1 verbinden lässt. Der Mittelsteg 2 verläuft hierbei durch den zuvor offenen Bereich des Durchbruchs 17, wobei die Flügelstege 3 mit ihrer dem Mittelsteg 2 zugewandten Oberfläche die Gewebepartien bzw. Wundränder 18 stabilisieren.

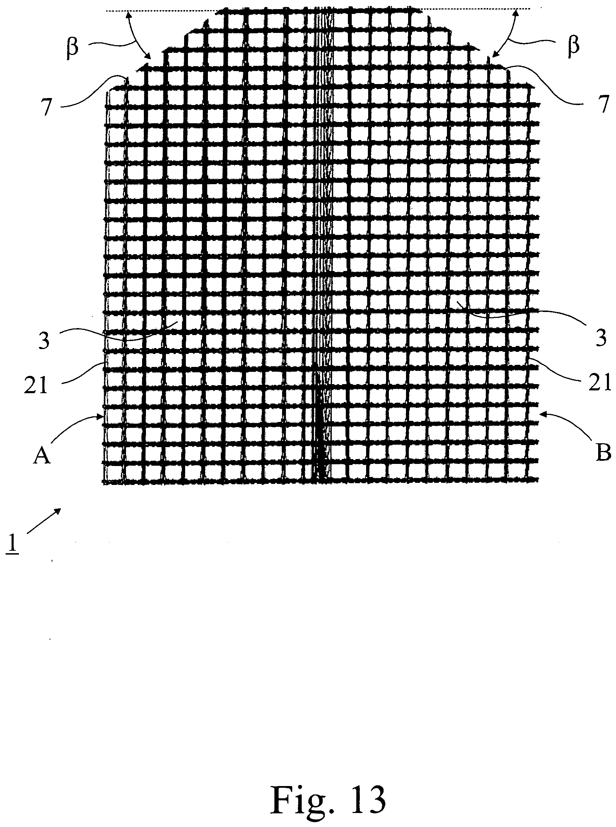

In den Figuren 13 bis 16 sind weitere alternative Ausführungsformen des Implantats 1 dargestellt. Figur 13 zeigt eine Draufsicht auf die Flügelstege 3 des Implantats 1, wobei die Pfeilung 7 der Flügelstege 3 geringer ausgebildet ist. Entsprechend nimmt der Winkel β zwischen dem abgeschrägten Abschnitt 5 der beiden Flügelstege 3 und der Stirnseite 4 einen kleineren Wert an als bei der Ausführungsform gemäß Figur 3. Der Winkel β der Pfeilung 5 kann an den jeweiligen Operationszweck geeignet angepasst sein.

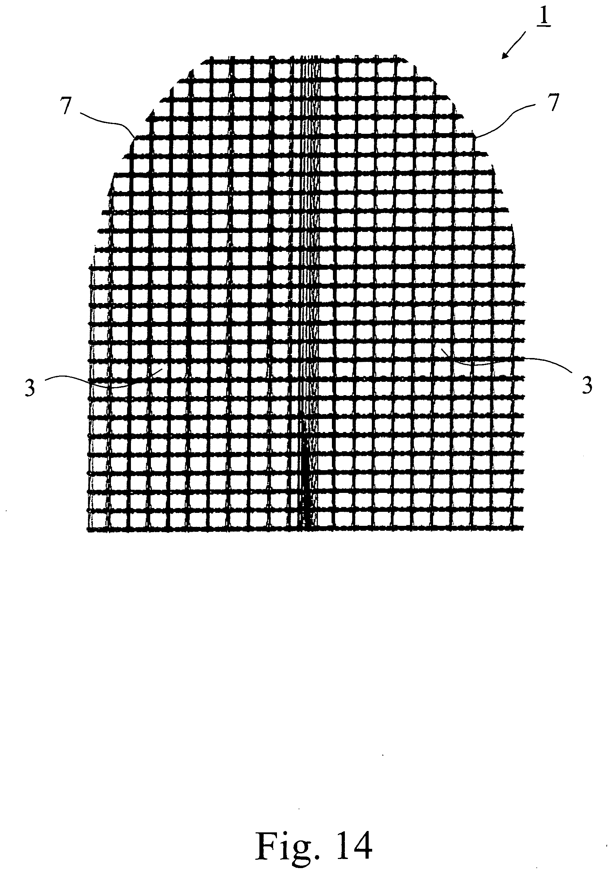

In Figur 14 ist eine Draufsicht auf eine weitere Ausführungsform des Implantats 1 gezeigt, bei der die Pfeilung 7 der beiden Flügelstege 3 konvex gerundet verläuft. Auch diese Ausgestaltung der Pfeilung kann in Anpassung an einen jeweiligen Operationszweck erfolgen.

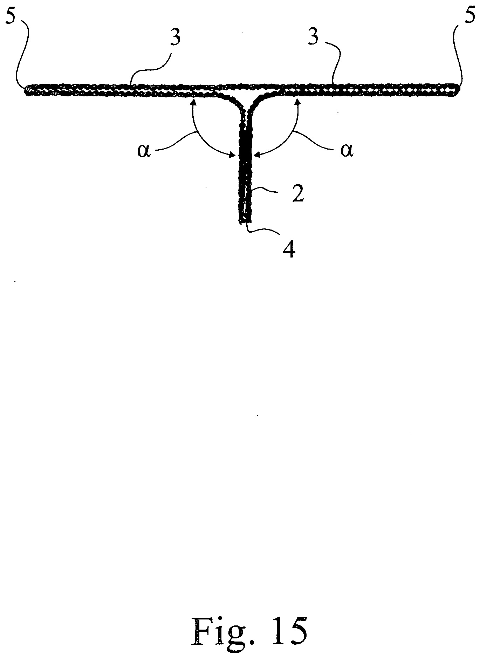

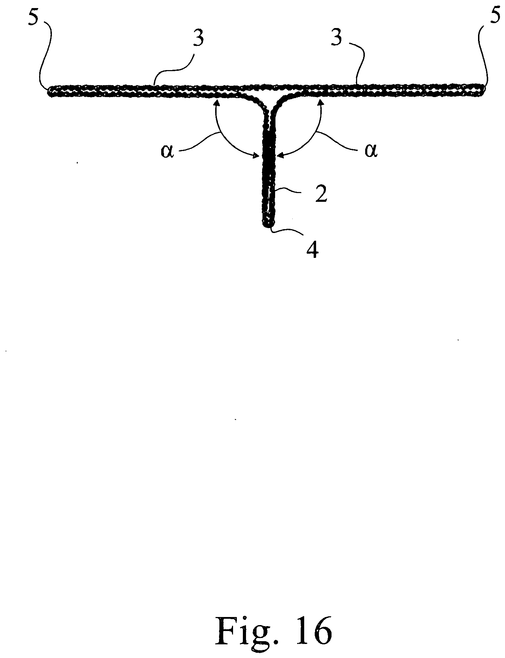

Figur 15 zeigt eine weitere Ausführungsform des Implantats 1, nämlich in einer Ansicht von hinten bzw. vorne. Hierin ist zu erkennen, dass der Winkel a zwischen dem Mittelsteg 2 und den jeweiligen Flügelstegen 3 im Wesentlichen 90 ° beträgt. Entsprechend hat das Implantat 1 bei dieser Ausführungsform einen T-förmigen Querschnitt. Die in Figur 15 gezeigte Ausführungsform kann durch eine Falttechnik gemäß Figur 5 hergestellt werden, wobei die äußeren Kanten 10 des flächigen Materials 8 im Bereich der unteren äußeren Kante 4 des Mittelstegs 2 geeignet zusammengefügt sind, analog zur Ausführungsform von Figur 2. In Abwandlung hierzu ist es bei einer weiteren Ausführungsform gemäß Figur 16 möglich, das Implantat 1 - in gleicher Weise wie bei der Ausführungsform von Figur 6 - aus einem Endlos-Schlauch zu falten, so dass auch die untere äußere Kante 4 des Mittelstegs 2 umgeschlagen bzw. gefaltet und dadurch gerundet ist.

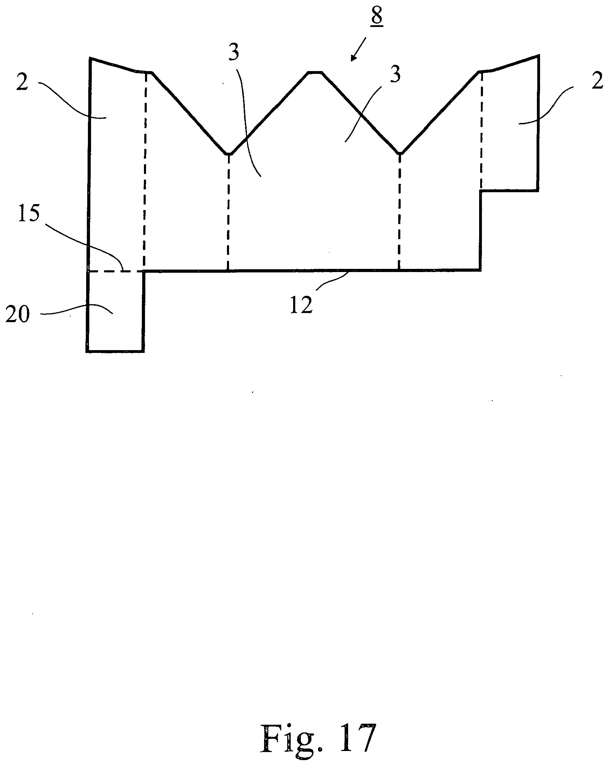

Figur 17 veranschaulicht einen weiteren Faltplan zur Herstellung des Implantats 1 nach einer weiteren Ausführungsform und zeigt eine Draufsicht auf das flächige Material 8 in seinem Ausgangszustand. In gleicher Weise wie in Figur 5 sind in der Darstellung von Figur 17 gestrichelte Linien gezeigt, entlang der das Material 8 zu falten ist, um das fertige Implantat 1 z.B. gemäß Fig. 1 bzw. Fig. 2 oder gemäß Fig. 15 zu bilden. Die wesentlichen Elemente des Implantats 1 in seinem Endzustand sind mit den dazugehörigen Bezugszeichen ebenfalls in der Figur 17 gezeigt.

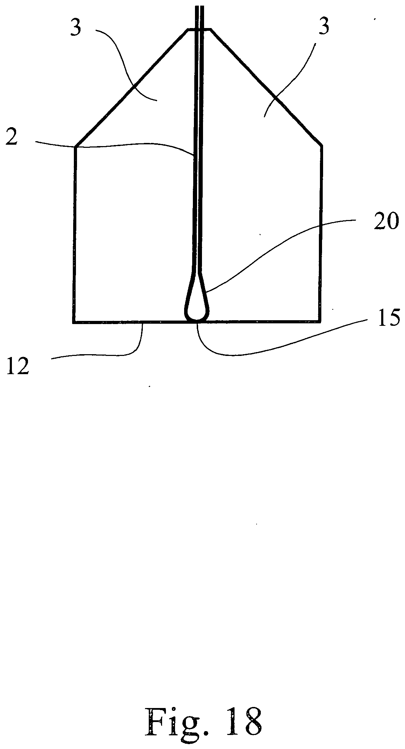

In seinem flächigen Ausgangszustand gemäß Figur 17 weist das Material 8 an einem Randbereich einen Lappen 20 auf. Dieser Lappen 20 ist im gefalteten Endzustand des Implantats 1 Teil des Mittelstegs 2, wobei der Lappen 20 entlang der hinteren äußeren Kante 15 auf einen mittigen Bereich des Mittelstegs 2 zurück umgeschlagen wird. Figur 18 zeigt das Implantat 1 in seinem gefalteten Endzustand in einer Ansicht von unten. Hierin ist zu erkennen, dass der Mittelsteg 2 an seiner hinteren äußeren Kante 15 durch das Umschlagen des Lappens 20 im Querschnitt gegen ein Ausfransen geschützt ist, und insbesondere auch gerundet ausgebildet ist. Das freie Ende des Lappens 20 wird im Endzustand des Implantats 1 auf dem Mittelsteg 2 in bekannter Weise fixiert, z. B. durch Kleben oder Thermofixieren.

Bei der Ausführungsform des Implantats 1 gemäß der Figuren 17 und 18 können die beiden Flügelstege 3 bezüglich des Mittelstegs 2 einen Winkel a einschließen, der entweder geringfügig größer als 90 ° (analog zur Darstellung von Fig. 2) oder im Wesentlichen 90 ° (analog zur Darstellung von Fig. 16) beträgt.

Das Implantat 1 lässt sich auch abweichend von den Faltplänen gemäß Figur 5 bzw. Figur 17 in anderer Weise geeignet zu einer dreidimensionalen Struktur falten. Desweiteren ist es möglich, dass bei dem Mittelsteg 2 auch dessen untere äußere Kante 4 und/oder dessen vordere äußere Kante 16 umgeschlagen wird/werden, und somit einen abgeschlossenen Rand aufweisen. In diesem Fall weist dann das Material 8 in seinem zweidimensionalen Ausgangszustand in den jeweiligen Wandbereichen entsprechend längere Lappen auf, die dann in gleicher Weise wie der Lappen 20 (Fig. 17, 18) umgeschlagen werden.

Die Draufsicht gemäß Fig. 3 bzw. Fig. 13 verdeutlicht für den mit einem Pfeil A markierten linken Flügelsteg 3, dass an dem abgeschlossenen Rand von dessen freier äußerer Kante 5 ein Einzelfilament bzw. ein Faden 21 im Wesentlichen entlang dieses abgeschlossenen Rands verläuft. Abweichend hierzu kann der abgeschlossene Rand einer freien äußeren Kante 5 des Flügelstegs 3 auch so ausgebildet sein, dass ein Einzelfilament 21 nicht direkt an dem abgeschlossenen Rand der äußeren freien Kante, sondern lediglich angrenzend und im Wesentlichen parallel dazu verläuft. Dies ist in der Darstellung von Fig. 3 und Fig. 13 für den jeweils rechten Flügelsteg 3 durch einen Pfeil B angedeutet. Der Verlauf eines solchen Einzelfilaments 21 entweder unmittelbar an dem abgeschlossenen Rand einer freien äußeren Kante, oder lediglich angrenzend dazu, kann die Formstabilität der freien äußeren Kante in gewünschter Weise beeinflussen. Abweichend von den Darstellungen der Fig. 3 und Fig. 13 versteht sich, dass ein Einzelfilament 21 bei beiden Flügelstegen 3 an deren jeweiligen freien äußeren Kanten 5 entweder wie bei Pfeil A gezeigt, d.h. direkt entlang des geschlossenen Rands, oder wie bei Pfeil B gezeigt, d.h. angrenzend an den geschlossenen Rand verlaufen kann.

Es versteht sich, dass die vorstehend erläuterten möglichen Ausführungsformen des Implantats 1 auch miteinander kombiniert werden können.

## Patentansprüche

1. Implantat (1), das dreidimensional ausgebildet ist und zumindest ein flächiges Lagenelement (2, 3) aus einem textilen Material mit zumindest einer freien äußeren Kante (4, 5) aufweist, wobei das Implantat (1) einen Mittelsteg (2) aufweist, von dem sich zumindest zwei zueinander entgegengesetzte Flügelstege (3) seitlich weg erstrecken, wobei die Flügelstege eine freie Kante aufweisen, **dadurch gekennzeichnet, dass** eine freie äußere Kante (4, 5) eines Lagenelements (2, 3) in zumindest einem Abschnitt einen abgeschlossenen Rand aufweist, an dem keine Faserenden oder dergleichen freiliegen, und dass die freie Kante der Flügelstege in zumindest einem Abschnitt einen abgeschlossenen Rand aufweist, und dass an dem abgeschlossenen Rand von der freien äußeren Kante (5) eines Flügelsteges (3) ein Faden (21) im Wesentlichen entlang dieses abgeschlossenen Rands verläuft, oder, dass ein Faden (21) nicht direkt an dem abgeschlossenen Rand der äußeren freien Kante (5), sondern lediglich angrenzend und im Wesentlichen parallel dazu verläuft und, dass zumindest ein Lagenelement (2, 3) in zumindest einem Abschnitt im Bereich einer freien äußeren Kante (4, 5) umgeschlagen ist.

2. Implantat (1) nach einem der vorhergehenden Ansprüche, **dadurch gekennzeichnet, dass** zumindest eine freie äußere Kante (4, 5) zumindest eines Lagenelements (2, 3) im Querschnitt gerundet ausgebildet ist.

3. Implantat (1) nach einem der vorhergehenden Ansprüche, **dadurch gekennzeichnet, dass** das Implantat (1) vollständig aus einem textilen Material (8) hergestellt ist, vorzugsweise, dass das textile Material netzartig ausgebildet ist.

4. Implantat (1) nach einem der vorhergehenden Ansprüche, **dadurch gekennzeichnet, dass** das Implantat aus einem textilen flächigen Material (8) gefaltet ist, vorzugsweise, dass das flächige Material (8) einstückig ist, weiter vorzugsweise, dass das zumindest eine flächige Lagenelement (2, 3) durch das Falten des Materials (8) eine Mehrzahl von übereinandergeschichteten Lagen aufweist, die aneinander insbesondere durch Thermofixieren und/oder Verkleben fixiert sind.

5. Implantat (1) nach einem der vorhergehenden Ansprüche, **dadurch gekennzeichnet, dass** ein Faden (21) des textilen Materials zumindest eines flächigen Lagenelements (2, 3) an dem abgeschlossenen Rand einer freien äußeren Kante (4, 5) angeordnet ist oder angrenzend dazu verläuft.

6. Implantat (1) nach einem der vorhergehenden Ansprüche, **dadurch gekennzeichnet, dass** zumindest ein flächiges Lagenelement (2, 3) von dem übrigen Teil des Implantats (1) flügelartig in Form eines Flügelstegs (3) abragt.

7. Implantat (1) nach einem der vorhergehenden Ansprüche, **dadurch gekennzeichnet, dass** das Implantat eine Mehrzahl von flächigen Lagenelementen (2, 3) aufweist, vorzugsweise, dass alle Lagenelemente (2, 3) zumindest eine freie äußere Kante (4, 5) mit einem abgeschlossenen Rand aufweisen, weiter vorzugsweise, dass die Mehrzahl von flächigen Lagenelementen in einem vorbestimmten Winkel (α) relativ zueinander positioniert sind, weiter vorzugsweise, dass zumindest zwei Lagenelemente (2, 3) relativ zueinander beweglich sind.

8. Implantat (1) nach einem der vorhergehenden Ansprüche, **dadurch gekennzeichnet, dass** das Implantat (1) im Querschnitt als T-Profil oder als H-Profil ausgebildet ist.

9. Implantat (1) nach Anspruch 8, **dadurch gekennzeichnet, dass** das Implantat derart gefaltet ist, dass zwei aneinander angrenzende Flügelstege (3) eine dem Mittelsteg (2) entgegengesetzten durchgehend verlaufende Oberseite (9) bilden.

10. Implantat (1) nach Anspruch 8 oder 9, **dadurch gekennzeichnet, dass** die untere äußere Kante (4) und/oder die hintere äußere Kante (15) und/oder die vordere äußere Kante (16) des Mittelstegs (2) umgeschlagen und vorzugsweise dadurch gerundet ist/sind, und/oder dass die untere äußere Kante (4) und/oder die hintere äußere Kante (15) und/oder die vordere äußere Kante (16) des Mittelstegs (2)mit einer Silikonschicht (14) versehen ist/sind.

11. Implantat (1) nach einem der Ansprüche 6 bis 10, **dadurch gekennzeichnet, dass** zumindest ein Flügelsteg (3) angrenzend an eine Stirnseite (4) des Implantats (1) gepfeilt ausgebildet ist, vorzugsweise, dass beide Flügelstege (3) an der gleichen Stirnseite des Implantats (1) gepfeilt ausgebildet sind, weiter vorzugsweise, dass die Pfeilung der Flügelstege (3) den gleichen Winkel (β) aufweist.

## Claims

1. Implant (1), being three-dimensional and having at least one planar sheet element (2, 3) of a textile material with at least one free outer edge (4, 5), the implant (1) having a central bar (2) from which at least two mutually opposite wing bars (3) extend laterally, the wing bars having a free edge, **characterized in that** a free outer edge (4, 5) of a sheet element (2, 3) has, in at least one portion, a closed border at which no fibre ends or the like lie exposed, and **in that** the free edge of the wing bars has, in at least one portion, a closed border, and **in that**, at the closed border of the free outer edge (5) of a wing bar (3), a thread (21) runs substantially along this closed border, or **in that** a thread (21) does not run directly at the closed border of the outer free edge (5) but only adjacent and substantially parallel thereto, and **in that** at least one sheet element (2, 3), in at least one portion, is turned back in the region of a free outer edge (4, 5).

2. Implant (1) according to one of the preceding claims, **characterized in that** that least one free outer edge (4, 5) of at least one sheet element (2, 3) has a rounded cross section.

3. Implant (1) according to one of the preceding claims, **characterized in that** the implant (1) is produced entirely from a textile material (8), preferably **in that** the textile material has a mesh-like configuration.

4. Implant (1) according to one of the preceding claims, **characterized in that** the implant is folded from a textile planar material (8), preferably **in that** the planar material (8) is on one piece, more preferably **in that** the at least one planar sheet element (2, 3) has, through the folding of the material (8), a plurality of sheets layered on top of one another, which are fixed to one another in particular by heat-setting and/or adhesive bonding.

5. Implant (1) according to one of the preceding claims, **characterized in that** a thread (21) of the textile material of at least of one planar sheet element (2, 3) is arranged at the closed border of a free outer edge (4, 5) or runs adjacent thereto.

6. Implant (1) according to one of the preceding claims, **characterized in that** at least one planar sheet element (2, 3) protrudes wing-like from the rest of the implant (1) in the form of a wing bar (3) .

7. Implant (1) according to one of the preceding claims, **characterized in that** the implant has a plurality of planar sheet elements (2, 3), preferably **in that** all of the sheet elements (2, 3) have at least one free outer edge (4, 5) with a closed border, more preferably **in that** the plurality of planar sheet elements are positioned at a predetermined angle (α) relative to one another, more preferably **in that** at least two sheet elements (2, 3) are movable relative to each other.

8. Implant (1) according to one of the preceding claims, **characterized in that** the implant (1) has, in cross section, the shape of a T profile or H profile.

9. Implant (1) according to Claim 8, **characterized in that** the implant is folded in such a way that two wing bars (3) adjoining each other form a continuous top face (9) opposite the central bar (2) .

10. Implant (1) according to Claim 8 or 9, **characterized in that** the lower outer edge (4) and/or the rear outer edge (15) and/or the front outer edge (16) of the central bar (2) are/is turned back and preferably thus rounded, and/or **in that** the lower outer edge (4) and/or the rear outer edge (15) and/or the front outer edge (16) of the central bar (2) are/is provided with a silicone coating (14).

11. Implant (1) according to one of Claims 6 to 10, **characterized in that** at least one wing bar (3), adjacent to an end face (4) of the implant (1), has an arrow shape, preferably **in that** both wing bars (3) are formed with an arrow shape at the same end face of the implant (1), more preferably in that the arrow shape of the wing bars (3) has the same angle (β).

## Revendications

1. Implant (1) réalisé en trois dimensions et présentant au moins un élément en couches plan (2, 3) composé d'un matériau textile muni d'au moins une bordure extérieure libre (4, 5), l'implant (1) présentant une nervure centrale (2) à partir de laquelle s'écartent latéralement au moins deux ailettes opposées (3), les ailettes présentant une bordure libre, **caractérisé en ce qu'**une bordure extérieure libre (4, 5) d'un élément en couches (2, 3) présente dans au moins une section une lisière fermée sans exposer des extrémités de fibre ou similaires, et **en ce que** la lisière libre des ailettes présente dans au moins une section une lisière fermée, et **en ce qu'**au niveau de la lisière fermée, à partir de la bordure extérieure libre (5) d'une ailette (3), s'étend un fil (21) substantiellement le long de cette lisière fermée, ou **en ce qu'**un fil (21) ne s'étend pas directement à la lisière fermée de la bordure libre extérieure (5) mais seulement à côté de et substantiellement en parallèle à celle-ci, et **en ce qu'**au moins un élément en couches (2, 3) est replié dans au moins une section au niveau d'une bordure extérieure libre (4, 5).

2. Implant (1) selon l'une quelconque des revendications précédentes, **caractérisé en ce qu'**au moins une bordure extérieure libre (4, 5) d'au moins un élément en couches (2, 3) est réalisée de manière arrondie en section transversale.

3. Implant (1) selon l'une quelconque des revendications précédentes, **caractérisé en ce que** l'implant (1) est fabriqué entièrement dans un matériau textile (8), de préférence **en ce que** le matériau textile est réalisé en treillis.

4. Implant (1) selon l'une quelconque des revendications précédentes, **caractérisé en ce que** l'implant est plié à partir d'un matériau plan textile (8), de préférence **en ce que** le matériau plan (8) est réalisé d'un seul tenant, de plus grande préférence que ledit au moins un élément en couches plan (2, 3) présente, suite au pliage du matériau (8), une pluralité de couches superposées qui sont fixées les unes aux autres, en particulier par thermofixage et/ou par collage.

5. Implant (1) selon l'une quelconque des revendications précédentes, **caractérisé en ce qu'**un fil (21) du matériau textile d'au moins un élément en couches plan (2, 3) est disposé à la lisière fermée d'une bordure extérieure libre (4, 5) ou s'étend de manière adjacente à celle-ci.

6. Implant (1) selon l'une quelconque des revendications précédentes, **caractérisé en ce qu'**au moins un élément en couches plan (2, 3) fait saillie à partir de la partie restante de l'implant (1) à la manière d'une aile sous la forme d'une ailette (3).

7. Implant (1) selon l'une quelconque des revendications précédentes, **caractérisé en ce que** l'implant présente une pluralité d'éléments en couches plans (2, 3), de préférence **en ce que** tous les éléments en couches (2, 3) présentent au moins une bordure extérieure libre (4, 5) avec une lisière fermée, de plus grande préférence **en ce que** la pluralité d'éléments en couches plans est positionnée selon un angle prédéterminé (α) les uns par rapport aux autres, de plus grande préférence **en ce qu'**au moins deux éléments en couches (2, 3) sont mobiles l'un par rapport à l'autre.

8. Implant (1) selon l'une quelconque des revendications précédentes, **caractérisé en ce que** l'implant (1) est réalisé en section transversale sous forme de profilé en T ou profilé en H.

9. Implant (1) selon la revendication 8, **caractérisé en ce que** l'implant est plié de telle sorte que deux ailettes (3) adjacentes l'une à l'autre forment une face supérieure (9) opposée à la nervure centrale (2) et s'étendant en continu.

10. Implant (1) selon la revendication 8 ou 9, **caractérisé en ce que** la bordure extérieure inférieure (4) et/ou la bordure extérieure arrière (15) et/ou la bordure extérieure avant (16) de la nervure centrale (2) est repliée et de préférence arrondie de ce fait, et/ou **en ce que** la bordure extérieure inférieure (4) et/ou la bordure extérieure arrière (15) et/ou la bordure extérieure avant (16) de la nervure centrale (2) sont munies d'une couche de silicone (14).

11. Implant (1) selon l'une quelconque des revendications 6 à 10, **caractérisé en ce qu'**au moins une ailette (3) est réalisée en forme de flèche à côté d'une face frontale (4) de l'implant (1), de préférence en ce que les deux ailettes (3) sont réalisées en forme de flèche sur la même face frontale de l'implant (1), de plus grande préférence **en ce que** la forme de flèche des ailettes (3) présente le même angle (β).
